# EUROPEAN PATENT SPECIFICATION

(11) **EP 0 920 447 B1**
(45) Date of publication and mention of the grant of the patent: **04.09.2002**
(21) Application number: 98913969.6
(22) Date of filing: 23.03.1998
(51) Int. Cl.: C07K 7/64

(54) **PROCESS OF PURIFICATION OF CYCLOSPORIN**
VERFAHREN ZUR REINIGUNG VOM CYCLOSPORIN
PROCEDE DE PURIFICATION DE CYCLOSPORINE

(30) Priority: 25.03.1997 HU 9700645
(43) Date of publication of application: 09.06.1999
(73) Proprietor: BIOGAL GYOGYSZERGYAR RT., 4042 Debrecen (HU)
(72) Inventor: KERI, Vilmos, H-4028 Debrecen (HU); NAGYNE, Arvai, Edit, H-4029 Debrecen (HU); DEAK, Lajos, H-4031 Debrecen (HU); MAKO, Györgyné, H-4031 Debrecen (HU); MISKOLCZY, Istvánné, H-4025 Debrecen (HU)
(74) Representative: HOFFMANN - EITLE
(86) International application number: HU9800029
(87) International publication number: WO98042734

(56) References cited:
- WO-A-94/16091
- WO-A-96/12031
- US-A- 5 382 655
- RÜEGGER ET AL.: 'CYCLOSPORIN A, EIN IMMUNOSUPPRESSIV WIRKSAMER PEPTIDMETABOLIT AUS TRYCHODERMA POLYSPORUM' HELVETICA CHIMICA ACTA vol. 59, no. 4, 1976, ZÜRICH, pages 1075 - 1092
- BUDAVARI ET AL.: 'THE MERCK INDEX, PAGES 464-465', 1996, MERCK, WHITEHOUSE STATION, NJ.

## Description

Object of the invention is a process for the chromatographic purification of cyclosporin A from a crude product containing cyclosporin complex by using a column filled with silica gel and by the application of multistep chromatography with a column filled with normal phase silica gel and by a solvent mixture containing toluene as the major component.

Cyclosporins are cyclic undecapeptides being N-methylated at several places and most of them having approved pharmacological effects. 25 members of this group of compounds have been known so far which are designated by letters A to Z. At first cyclosporin A was separated among them, which is a natural material and was isolated from the culture broth of Tolypocladium inflatum Gams strain (Helv. Chini. Acta 59. 1075/1976/). First this compound became known as a slight antifungal antibiotic, later attracted the attention to its immunosuppressive effect (J.F. Borel et al: Immunology 32 1017/1977/). For this reason it was mainly used in organ transplantation from other persons (lung, heart, kidney, bone marrow, skin). Pharmacological examinations proved that it inhibits both the humoral and cellular immune responses by hindering the proliferation of T-cell and interrupting the synthesis of interleukin-2. Later it was cleared out, that it is efficient in different type of autoimmune and inflammatory diseases, such as in autoimmune Haematological diseases, ulcerous colitis, Graves-illness, multiple sclerosis, psoriasis, rheumatic arthritis as well. Further experiments were done to cure infections caused by protozoa and tumours too. The importance of this group is indicated by the fact, that number of synthetic related compounds can be prepared by building in different amino acids and substitutes (e.g. EP 56782, CH 630062 and EP 29122 patents).

Major part of the cyclosporins are prepared by fermentation. As examples the cultures of the microorganisms Cylindrocarpon Lucidum Booth (patent specification No. CH 589,716); Trichoderma polysporum Rifai (patent specification No. CH 603,790); Tolypocladium varium (patent specification No. HU 201,577) are used. At the end of the fermentation cyclosporin complex is formed which may contain other impurities too (ingredients of culture media, antifoaming agent, metabolites, etc.) depending on the character of the process.

Generally the product is isolated from the broth by extraction processes. This can be done by separating the mycelium from the broth by centrifugation or filtration, then dissolving the active ingredient from the mycelium by methanol or acetone and extracting the filtrate by water-immiscible solvents. An other known execution method is a process without filtration, using whole broth extraction with water-immiscible organic solvent. Solvent content of the organic phase is evaporated by vacuum distillation. However during the organic solvent extraction the compounds having lipid characters are also transferred into the organic phase, which cause difficulties in the further purification. In order to separate these compounds there are known processes (e.g. Swiss patent specification No. 589,716 or published German patent application No. 2455859) where after removing the extracting solvent the residue is dissolved in a mixture of methanol-water and then is extracted several times by the same volume of petroleum ether. Petroleum ether portions are combined and cyclosporins are recovered from them by a mixture of methanol-water. The active substance is transferred by multi-extraction from the combined methanol-water phase into ethylene chloride, which is then washed with water and evaporated to dryness. The crude product prepared by the above method and containing cyclosporins can be purified more efficiently by one of the chromatographic methods.

According to a method described in U.S. Pat. No. 4,117,118 the cyclosporin mixture is transferred first to a Sephadex LH-20 column and eluted with methanol, then it is eluted successively in an alumina column with a mixture of toluene and ethyl acetate (15%), and in a silica gel column with a mixture of chloroform and methanol (2%). Despite of the repeated chromatography the resulting product is not pure, but it is a mixture of cyclosporin A and B.

A similar chromatographic process is disclosed among others in the U.S. Pat. No. 4,215,199, where a rough-cleaning is with a 98:2% v/v mixture of chloroform and methanol on a silica gel column. The eluate is then evaporated to dryness. The residue is dissolved in methanol and is subjected to chromatography in a Sephadex LH-20 column using methanol as eluent. The eluate fractions are evaporated to dryness, then the residue is dissolved in 98:2% v/v mixture of chloroform and methanol. It is subjected again to a silica gel chromatography. Cyclosporin A appears first in the eluate. This and the consecutive fractions are separated, and the pure components are obtained by evaporating the elutes.

According to the German Pat. No. DD 298276 the oily crude product is dissolved in small quantity of chloroform, then subjected to chromalography in an alumina column with chloroform. The fractions containing cyclosporin A are evaporated in vacuum, dissolved in chloroform, subjected to a similar column and then eluted by chloroform. Fractions containing the active substance are evaporated in vacuum again. Hexane is added to the residue and the cyclosporin A is crystallised. Product is washed by hexane then dried and finally recrystallised from a mixture of ether and hexane or acetone.

According to the Hungarian Pat. No. 201577 the crude product obtained after evaporation can be cleaned on a silica gel column by elution with a mixture of chloroform and methanol gradually increasing concentration of methanol. The process is started by pure chloroform and continued by increasing in 0.5 vol% steps the methanol in the eluate. Cyclosporin A is eluted by 2 vol%, cyclosporin B by 2.5 vol%, cyclosporin C by 3 vol% methanol containing chloroform from the column. Components are obtained by evaporating the fractions.

Processes mentioned above describe mainly cyclosporin fermentation procedures, where the first aim of the purification steps is to identify the product obtained. Thus the product is isolated only in a small quantity and only the physical and chemical characteristics are given without publishing data relating to the purity of the product and the quantities of the impurities.

Rüeger and Co. /Helv.Chim. Acta 59(4), p.1075-92 (1976)/ isolated small quantities of pure cyclosporin A and C by repeated chromatographies and by other purification steps for identification and structure analysis. According to this article the crude product received from the fermentation of Trichoderma polysporum Rifai containing mainly cyclosporins A and C is defatted with methanol and petroleum ether. After evaporation the residue is dissolved in chloroform and is subjected to chromatography by gradient elution with 98.5:1.5 v/v mixture of chloroform and methanol as eluent. Pure crystalline cyclosporin A is obtained by further chromatography. The fraction containing cyclosporin A is dissolved in methanol and is subjected to chromatography in a Sephadex LH-20 column by using methanol as eluent. Peak fractions are evaporated, dissolved in toluene and subjected to chromatography in a column packed with aluminium oxide, using toluene as eluent, in the presence of an increasing concentration of acetic acid. The crystalline product is obtained after the evaporation of fractions and a treatment with activated carbon in an alcoholic solution.

A purification process feasible in industrial scale is described in the U.S. Pat. No. 5382655. According to the process the crude product containing different cyclosporin components is subjected to heat treatment prior to chromatography on silica gel column by the mixtures of chloroform-dichloromethane-ethanol and chloroform-ethyl acetate-ethanol. The product obtained is subjected to further chromatography and recrystallization, which resulted in a pure quality of product good for injection production.

Purification of the crude product containing the mixture of cyclosporins is very difficult, since the impurities having similar chemical structures are very similar in chromatographic characteristics to cyclosporin A as the main product. As the previously described processes prove, that regardless of the employed solvent mixture and because of the overlapping of the chromatographic peaks further chromatographic or other purification steps have to be carried out for obtaining certain components in pure form. The purification processes known so far are generally characterised by not only the fact, that their solvent requirement is considerable large, but the application of 3-4 different type of solvents or solvent mixtures and 2-3 types of column fillings are needed as well. As a consequence of these facts several type of chromatographic technics and regeneration methods are required within a process, which make difficulties in the development of a simple constructed and uniformly manageable economic industrial scale technology.

From environment protection point of view further problems are raised by the application of chlorinated hydrocarbons, since more and more countries have been making efforts for the limitation of their usage.

Regarding the filling materials of columns the application of aluminium oxide filling for industrial purposes is very doubtful, because as a consequence of its small specific surface the loading capacity and the separation ability is very small. Furthermore it is not advantageous for industrial purposes, because the aluminium oxide filling is rigid and easily fragile, thus it needs a special equipment and technology. Those can not be used in frequently emptied stainless steel columns generally used in the chemical industry.

Sephadex type fillings are very expensive ones, and in the case of the cyclosporin complex their efficiencies are very limited, since the sizes of molecules are very near to each others.

The object of the present invention is the development of an easily applicable chromatographic purification method in industrial scale which is suitable to manufacture the cyclosporin A ingredient containing much less impurities so far thus using it in medical practice in a safer way.

Our aim was to develop such a chromatographic purification technology, which requires only one type of solvent mixture and one type of column filling.

Because of its advantageous characters - high specific surface, high pore size, favourable sorption ability, easy handling and relatively low price - silica gel was used as a column filling. An ideal solvent mixture and a method had to be selected to this filling which is suitable to separate the cyclosporin components with high selectivity.

As a result of our experiments we recognised that our aim can be realised by multistep chromatography on silica gel column using a solvent mixture as an eluent which main component is toluene. Surprisingly it was found that even cyclosporin U and L components being the nearest to cyclosporin A can be separated by a three stages chromatography on silica gel column using toluene containing acetone as an eluent. These components differ from cyclosporin A only in a methyl group.

According to the first example of the application No. WO 94/16091 a solvent mixture of toluene and acetone is also used in a single stage. The product obtained is recrystallized from a solvent mixture of ether and hexane. (Yield: 66,6 %) The optical rotation of the product is sufficient, but its melting point is remarkably lower than that described in the literature, which indicates that the product is not pure. Thus, even recrystallization from a solvent will yield product of poor purity and a low yield.

Process according to the present invention is suitable to separate the most frequent impurities such as cyclosporins B and C being at present the largest quantities and moreover cyclosporin D, U and L components being at present in trace quantities. Cyclosporins B and C content of cyclosporin A end product obtained this way is less then 0.02 vol%, while cyclosporins L, U and D content is below 0.05 vol%.

The object of our invention is an improved process for the purification of cyclosporin A from a crude product containing cyclosporin complex, which also enables the production of extremely pure cyclosporin A in large scales, with a chromatographic method on silica gel column using multistep chromatography with a solvent mixture containing toluene as the main component. Another new feature of the process is that we apply extremely high column load. In the conventional chromatographic practice the extent of column load amounts to not more than 5-10 % of the column charge, and this value is lower for cyclosporins.

The multistep chromalography, the solvent mixture containing toluene as the main component and the high column load are interrelated and the desired result can only be achieved by their combined use. Therefore all three of above properties should be used simultaneously so as to obtain the extremely pure product.

According to the present invention preferably 2-4 chromatographic steps are applied subsequently and more expediently three steps.

The overload of the column is the highest in the first step of chromatography. The definite separation of the active substance and the impurities allows the use of high column load in the subsequent two steps of chromatography as well.

For the purification it is favourable to use a solvent mixture of toluene-acetone, which contains at most 30 vol% acetone.

According to an other favourable method a solvent mixture of toluene-ethyl acetate is used in which the ethyl acetate concentration is below 35 vol%.

In the purification process at least one time it is favourable to use gradient elution.

According to our experiments it was found that for the purification of the cyclosporin complex processed in our case is favourable to use 10-30 vol% and more favourable to use 13-18 vol% acetone and 10-35 vol% or 15-20 vol% ethyl acetate.

According to a possible method of the present invention the elution is done in the case of the three steps chromatography with toluene containing 15 vol% acetone or 18 vol% ethyl acetate. In the first step the major part of cyclosporin C is separated, in the second step the larger part of cyclosporin B , L and U components are removed, and finally in the third step the quantities of L and U components and other unidentified impurities can be reduced below 0.05 vol%. In the first step the cyclosporin A loss is minimal, however in the pre-fractions of the second and third steps a considerable quantity of cyclosporin A is removed together with the cyclosporin D component being very close to cyclosporin A. This cyclosporin A can be recovered in a very pure form in the fourth step.

For comparison in the table below the impurity profiles of cyclosporin A USP standard, the cyclosporin A active ingredient from SANDIMMUN® injection and also the data of the cyclosporin A product prepared by the present invention are given.

| **USP standardSANDIMMUN® inj.Product according to the Example 1** | | | |
|---|---|---|---|
| **Impurities** | **vol%** | **vol%** | **vol%** |
| Cyclosporin C | - | 0.17 | 0.05 |
| Cyclosporin B | - | 0.21 | 0.05 |
| Unknown | 0.09 | 0.35 | 0.05 |
| Cyclosporin L | 0.05 | 0.35 | 0.05 |
| Cyclosporin U | - | - | 0.05 |
| Cyclosporin D | 0.12 | - | 0.05 |

From the data it can be seen, that the quality of the cyclosporin A obtained by the present invention considerably exceeds the parameters of the SANDIMMUN® injection, moreover overfulfils even the USP requirements too.

Process according to the present invention is applicable for the purification of crude product both in small and large scales too.

Beside the fact that the process according to the present invention is able to prepare pure cyclosporin A, it has some not negligible technological advantages too. Since only one type of technological method (chromatography) is used, the purification process is manageable uniformly, moreover it is repeatable and can be converted into a continuous process. Furthermore there is a special advantage, that since only one type of solvent mixture is used in the three chromatographic steps, the regeneration of both the columns and the solvents became more simple.

A further special benefit of the process is that the first step of chromatography the highly binding impurities remain on the column, which greatly facilitates the regeneration of column charges in the subsequent two steps.

Invention is introduced by the examples below without limiting the protection demand only to them.

In a comparative example (Example 4) it is presented, that with the application of a solvent mixture of dichloromethane-acetone in 3 steps chromatography generally used so far in the known processes, could not be produced a final product in a similar purity.

### Example 1

### Purification of cyclosporin crude product with three steps chromatography with the application of a solvent mixture of toluene-acetone

Quality of the starting cyclosporin crude product:
Cyclosporin A content 60.9 vol%
Cyclosporin B content 11.2 vol%
Cyclosporin C content 8.3 vol%
Cyclosporin L content 1.79 vol%
Cyclosporin U content 1 .58 vol%
Cyclosporin D content 1.25 vol%

### 1st step

Chromatography is done with two chromatographic columns connected in series, each one is 8 litre with jacket, diameter 10 cm, length 100 cm. Each of the two columns contains 3.95-3.95 kg Merck type Kieselgel silica gel with grain size 0.04-0.063 mm. In the first chromatography both columns contain fresh silica gel. In the case of the next chromatography the first column is separated and a second column is connected to it containing fresh silica gel. Further on for each chromatography only one new column is used.

### Preparation of the crude product

4.1 kg with a purity of 60.9 vol% crude product is loaded to a pair of columns connected in series being dissolved previously in 15 litre toluene. 19 litre solution is received which is subjected to the top of the column through a filter with a 2.4 l/h feeding rate. After loading the material is eluated with a solvent mixture of 13:87 vol% acetone-toluene until the volume of the effluent at the bottom of the second column is 39 litre. The cyclosporin content of the effluent is analysed by TLC. Fractions do not containing cyclosporin are collected as waste. 28 litre of the effluent after appearing the cyclosporin is considered as the main fraction. Dry material content of the intermediate 1 obtained this way is 3.23 kg.

| | |
|---|---|
| Quality | cyclosporin A 75 vol% |
| | cyclosporin B 10.1 vol% |
| | cyclosporin C 1.6 vol% |
| | cyclosporin L 1.7 vol% |
| | cyclosporin U 1.5 vol% |
| | cyclosporin D 1.3 vol% |
| Yield calculated to cyclosporin A | 97 % |

### 2nd step

Separation is done in a 1 meter length jacketed 8 litre column. Column contains Merck Kieselgel 60 silica gel (0.015-0.040 mm). Mass of the filling is 3.95 kg.
About 3 litre volume and 370 g dry material containing intermediate I solution obtained in the first step is subjected to the column with 2.4 l/h feeding rate, then it is washed with 1 litre toluene.

After loading the material the column is eluated with the mixtures of 10 litre 15:85 vol% acetone:toluene and then with 20 litre 25:75 vol% acetone:toluene solutions.
Flow rate of the solvent till 17 litre fraction is 2.4 l/h, then from 18 litre fraction 5 l/h. Fractions are analysed by TLC. 1-11 litre fractions are waste, fractions 12-19 litre are considered as critical I fractions and samples are taken from them. Impurity profiles of them are analysed by HPLC and those are handled as pre-fractions or combined with the main fractions. This way 80 g dry material containing pre-fraction can be received which is then evaporated to dryness. 20-25 litre fractions are combined as main fraction. 26-31 litre fractions are considered as critical fractions and after analysis those are combined with the main fraction or handled as post-fraction.
Main fraction obtained the above way is evaporated to dryness in a film evaporator mounted with an oscillating stirrer. 234 g of intermediate 11 is obtained with a yield of 80 % and with a quality of the following:
cyclosporin A 95 vol%
cyclosporin U 1.2 vol%
cyclosporin L 0.7 vol%
cyclosporin B <0.1 vol%
cyclosporin D 0.5 vol%
cyclosporin C 0.1 vol%

### 3rd step

Chromatography is done on the columns with the same construction and geometric sizes described in the 1st and 2nd steps.

1.7 litre toluene solution containing 220 g dry material is prepared from the intermediate II obtained in the second step and subjected to the top of the column with 2.4 l/h feeding rate, then it is washed with 1 litre toluene.

Column is elualed with a mixture of 20 litre 15:85 vol% acetone:toluene and then the cyclosporin is eluated with a mixture of 20 litre 25:75 vol% acetone:toluene.
Flow rate of the elution till 31 litre fraction is 2,4 l/h, then from 32 litre fraction 5 l/h. 1-18 litre fractions are waste, fractions 19-23 litre are pre-fractions and considered as critical I fractions. Samples are taken from them in order to analyse the dry material content and the impurity profiles by HPLC. After analysis those are handled as pre-fractions or combined with the main fractions. 29-38 litre fractions are combined as main fraction. 39-41 litre fractions are collected in 1 litre portions and considered as critical fractions II. After analysis those are combined with the main fraction or handled as post-fraction.
As a result of the fractions collection after evaporation to dryness 70 g pre-fraction can be received.
Main fractions are combined and after evaporation to dryness 157 g pure cyclosporin is obtained with a yield of 75 %. Quality of the product is the following:
cyclosporin A 99.6 vol%
cyclosporin L <0.05 vol%
cyclosporin U <0.05 vol%
cyclosporin D <0.05 vol%
cyclosporin B <0.02 vol%
cyclosporin C <0.02 vol%

### Example 2

### Chromatographic purification of cyclosporin crude product ou stationary bed four steps silica gel column with the application of solvent mixtures of toluene-acetone or toluene-ethyl acetate

Cyclosporin crude product is purified with a three steps chromatography described in Example 1. Pre-fractions received in the three steps chromatography are purified in the fourth step in stationary bed with a solvent mixture of toluene-ethyl acetate.

### 4th step

Construction and geometric sizes of chromatographic column is the same as described in Example 1. Column contains Merck Kieselgel 60 type silica gel (0.015-0.040 mm) as it is written in Example 1.
Column is subjected by a concentrate received from pre-fraction in the quantity of 260 g material dissolved in 2.5 litre toluene with a feeding rate of 2.4 l/h.
cyclosporin A content 80.6 vol%
cyclosporin D content 4.2 vol%

Subjected sample is washed with 1 litre toluene, then the column is eluated with a mixture of 20 litre 17:83 vol% ethyl acetate:toluene by a flow rate of 2.4 l/h, then the elution is continued with a mixture of 40 litre 28:72 vol% ethyl acetate:toluene.
In the course of fractions collection the 1-19 litre fractions are waste. After HPLC analysis the 20-25 litre fractions are waste or combined with the main fraction. 26-35 litre fractions are collected as main fraction. After sampling and HPLC analysis the 36-42 litre fractions either combined with the main fraction or handed as waste. 23-42 litre collected main fraction described above is evaporated to dryness. This way 195 g pure cyclosporin A is obtained containing 99.6 vol% active ingredient with a yield of 75 % and with a quality of the following:
cyclosporin A 99.6 vol%
cyclosporin D <0.05 vol%
cyclosporin U -
cyclosporin L -

### Example 3

### Chromatographic purification of cyclosporin crude product on stationary bed two steps silica gel column with the application of a solvent mixture of toluene-acetone

Cyclosporin crude product is purified according to the same process described in the 1st step of Example 1 obtaining cyclosporin intermediate I with the same quality. Further on the process is the following:

### 2nd step

Column is subjected by 3 litre intermediate I containing 370 g dried material with a feeding rate of 2.4 l/h, then the subjected sample is washed with 1 litre toluene.

After loading the column is eluated with a mixture of 10 litre 15:85 vol% acetone:toluene, then the elution is continued with a mixture of 20 litre 25:75 vol% acetone:toluene.
Flow rate of the solvent is 2.4 l/h till 17 litre and 5 l/h from 18 litre.
According to TLC and HPLC detection 1-11 litre fractions are waste, 12-20 fractions are pre-fractions, 21-24 fractions are considered as the main fractions and fractions from 25 till the end are handled as post-fractions. Washing acetone fractions are waste.

After fractionating the collected main fractions are combined and evaporated to dryness obtaining 114 g cyclosporin A with a yield of 41 % and with as good quality of product as is written in Example 1.

### Example 4

### Comparative example for the purification of crude product with 3 steps chromatography with the application of a solvent mixture of dichloromethane-acetone

Quality of the starting cyclosporin crude product (the same as used in Example 1):
Cyclosporin A content 60.9 vol%
Cyclosporin B content 11.2 vol%
Cyclosporin C content 8.3 vol%
Cyclosporin L content 1.79 vol%
Cyclosporin U content 1.58 vol%
Cyclosporin D content 1.25 vol%

### 1st step

Chromatographic equipment and the filling is the same as described in Example 1. One pair of column connected in series is subjected with 4.1 kg crude product with the purity of 60.9 % in 15 litre dichloromethane solution.

After loading the sample the column is eluated with dichloromethane with a flow rate of 2.4 l/h till collecting 35 litre of effluent.
1-10 litre fractions are waste, while 11-35 litre fractions are considered as main fractions. Dried material content of intermediate I obtained this way is 2.9 kg, active ingredient content is 75 vol%.

### 2nd step

Chromatographic equipment and the filling is the same as described in Example 1.

Column is subjected at the top by 3 litre intermediate I containing 350 g dried material with a feeding rate of 2.4 l/h. Elution is done with a mixture of 10 litre acetone:dichloromethane 1:9 vol. rate, then continued with a mixture of 25 litre acetone:dichloromethane 2:8 vol. rate and finished with acetone 2.4 l/h rate.
According to TLC analysis the volume of the pre-fraction was 13 litre, the volume of the main fraction was 22 litre, and the volume of the post-fraction was 11 litre. 22 litre of main fraction was evaporated to dryness and as a consequence 220 g intermediate II product was obtained with the purity of 91 %.

### 3rd step

Chromatographic equipment and the filling is the same as described in Example 1. Filling is loaded by 220 g intermediate II feeding the dichloromethane concentrate with a rate of 2.4 l/h on the column. Elution is done with a mixture of 20 litre acetone:dichloromethane 1:9 vol. rate, then continued with a mixture of 30 litre acetone:dichloromethane 2:8 vol. rate and finished with acetone 2.4 l/h rate.
First 26 litre fractions are considered as pre-fractions, then 26 litre main fraction is collected and finally 11 litre post-fractions are taken.
26 litre of main fraction was evaporated to dryness and as a consequence 140 g intermediate III product was obtained with the following quality:
Cyclosporin A 98.6 vol%
Cyclosporin U 0.6 vol%
Cyclosporin D 0.3 vol%
Cyclosporin L 0.2 vol%
Cyclosporin B 0.1 vol%
Cyclosporin C 0.1 vol%

## Claims

1. A purification process for the preparation of cyclosporin A in high purity from cyclosporin complex containing crude product with a chromatographic method on silica gel column wherein multistep chromatography is used with a solvent mixture containing toluene as the main component.

2. A process of claim 1, wherein 2-4 chromatographic steps are carried out subsequently.

3. A process of claim 1, wherein 3 chromatographic steps are carried out subsequently.

4. A process of claim 1, wherein toluene-acetone is used as a solvent mixture.

5. A process of claim 4, wherein toluene containing at most 30% acetone is used.

6. A process of claim 1, wherein a solvent mixture of toluene-ethyl acetate is used.

7. A process of claim 6, wherein toluene containing at most 35 vol% ethyl acetate is used.

8. A process of claim 1, wherein gradient elution is used in the case of at least one chromatographic step.

9. High purity cyclosporin A, wherein its cyclosporin L, cyclosporin U and cyclosporin D content is less then 0.05 vol% and its cyclosporins B and C content is less then 0.02 vol%.

10. An industrial scale process for the purification of cyclosporin A from cyclosporin complex containing crude product with a chromatographic method on silica gel column wherein multistep chromatography is carried out on the column containing normal phase silica gel with a solvent mixture containing toluene as the main component.

11. Any process of claims 1 or 10, wherein the cyclosporin complex used as the starting material of the chromatography is heated up to 80-120 °C prior to chromatography.

12. Any process of claims 1 or 10, wherein toluene containing 10-30% acetone is used.

13. Any process of claims 1 or 10, wherein toluene containing 10-35 vol% ethyl acetate is used.

## Patentansprüche

1. Reinigungsprozeß zur Herstellung von Cyclosporin A in hoher Reinheit aus Cyclosporin-Komplex, der Rohprodukt enthält, mit einem chromatographischen Verfahren an einer Silicagel-Säule, wobei Mehrschrittchromatographie mit einer Lösungsmittelmischung, die Toluol als Hauptkomponente enthält, verwendet wird.

2. Prozeß von Anspruch 1, wobei anschließend 2 bis 4 chromatographische Schritte durchgeführt werden.

3. Prozeß von Anspruch 1, wobei anschließend 3 chromatographische Schritte durchgeführt werden.

4. Prozeß von Anspruch 1, wobei Toluol-Aceton als Lösungsmittelmischung verwendet wird.

5. Prozeß von Anspruch 4, wobei Toluol verwendet wird, das höchstens 30 % Aceton enthält.

6. Prozeß von Anspruch 1, wobei eine Lösungsmittelmischung aus Toluol-Ethylacetat verwendet wird.

7. Prozeß von Anspruch 6, wobei Toluol verwendet wird, das höchstens 35 Vol.-% Ethylacetat enthält.

8. Prozeß von Anspruch 1, wobei im Fall mindestens einer chromatographischen Stufe Gradientenelution verwendet wird.

9. Cyclosporin A mit hoher Reinheit, wobei sein Cyclosporin L-, Cyclosporin U- und Cyclosporin D-Gehalt weniger als 0,05 Vol.-% und sein Cyclosporin B- und C-Gehalt weniger als 0,02 Vol.-% ist.

10. Prozeß in industriellem Maßstab für die Reinigung von Cyclosporin A aus Cyclosporin-Komplex, der Rohprodukt enthält, mit einem chromatographischen Verfahren an einer Silicagel-Säule, wobei an der Säule, die Normalphasen-Silicagel enthält, Mehrschrittchromatographie mit einer Lösungsmittelmischung, die Toluol als Hauptkomponente enthält, durchgeführt wird.

11. Prozeß der Ansprüche 1 oder 10, wobei der als Ausgangsstoff der Chromatographie verwendete Cyclosporin-Komplex vor der Chromatographie auf 80 bis 120°C erwärmt wird.

12. Prozeß von Anspruch 1 oder 10, wobei Toluol verwendet wird, das 10 bis 30 % Aceton enthält.

13. Prozeß von Anspruch 1 oder 10, wobei Toluol verwendet wird, das 10 bis 35 Vol.-% Ethylacetat enthält.

## Revendications

1. Procédé de purification pour la préparation d'une cyclosporine A de grande pureté, à partir d'un complexe de cyclosporine contenant du produit brut, à l'aide d'une technique chromatographique appliquée sur colonne de gel de silice, dans laquelle une chromatographie multi-étape est mise en oeuvre avec un mélange solvant contenant du toluène en tant que composant principal.

2. Procédé selon la revendication 1, dans lequel 2 à 4 étapes de chromatographie sont effectuées ultérieurement.

3. Procédé selon la revendication 1, dans lequel 3 étapes de chromatographie sont effectuées ultérieurement.

4. Procédé selon la revendication 1, dans lequel le mélange toluène/acétone est utilisé comme solvant mixte.

5. Procédé selon la revendication 4, dans lequel il est utilisé du toluène contenant au maximum 30 % d'acétone.

6. Procédé selon la revendication 1, dans lequel il est utilisé un mélange solvant toluène/acétate d'éthyle.

7. Procédé selon la revendication 6, dans lequel il est utilisé du toluène contenant au maximum 35 % en volume, d'acétate d'éthyle.

8. Procédé selon la revendication 1, dans lequel un gradient d'élution est mis en oeuvre, au minimum dans le cas d'une étape de chromatographie.

9. Cyclosporine A de grande pureté dont la teneur en cyclosporine L, cyclosporine U et cyclosporine D est inférieure à 0,05 % en volume, et la teneur en cyclosporines B et C est inférieure à 0,02 % en volume.

10. Procédé à l'échelle industrielle, pour la purification de la cyclosporine A à partir d'un complexe de cyclosporine contenant du produit brut, à l'aide d'une technique chromatographique appliquée sur colonne de gel de silice, dans laquelle une chromatogra-phie multi-étape est mise en oeuvre sur une colonne contenant une phase gel de silice normale, avec un mélange solvant contenant du toluène en tant que composant principal.

11. Procédé quelconque selon la revendications 1 ou la revendication 10, dans lequel le complexe de cyclosporine employé comme produit de départ pour la chromatographie est chauffé jusqu'à 80-120°C avant ladite chromatographie.

12. Procédé quelconque selon la revendications 1 ou la revendication 10, dans lequel il est utilisé du toluène contenant 10 à 30 % d'acétone.

13. Procédé quelconque selon la revendications 1 ou la revendication 10, dans lequel il est utilisé du toluène contenant 10 à 35 % d'acétate d'éthyle.
